(19) Europäisches Patentamt
European Patent Office
Office européen des brevets

(11) EP 2 950 361 B1

(12) EUROPEAN PATENT SPECIFICATION

(45) Date of publication and mention of the grant of the patent:
**17.04.2019 Bulletin 2019/16**

(21) Application number: **14743884.0**

(22) Date of filing: **27.01.2014**

(51) Int Cl.:
***H01L 51/50*** *(2006.01)*
***C07C 211/54*** *(2006.01)*
***H05B 33/10*** *(2006.01)*
***H01L 51/00*** *(2006.01)*
***C09D 5/24*** *(2006.01)*
***C09D 179/02*** *(2006.01)*
***C08G 73/02*** *(2006.01)*

(86) International application number:
**PCT/JP2014/051619**

(87) International publication number:
**WO 2014/115865 (31.07.2014 Gazette 2014/31)**

# (54) CHARGE-TRANSPORTING VARNISH

LADUNGSTRANSPORTLACK

VERNIS À TRANSPORT DE CHARGE

(84) Designated Contracting States:
**DE NL**

(30) Priority: **28.01.2013 JP 2013012844**

(43) Date of publication of application:
**02.12.2015 Bulletin 2015/49**

(73) Proprietor: **Nissan Chemical Corporation**
**Tokyo (JP)**

(72) Inventors:
- **NAKAIE Naoki**
**Funabashi-shi**
**Chiba 274-0052 (JP)**
- **TAKAYAMA Yuki**
**Funabashi-shi**
**Chiba 274-8507 (JP)**

(74) Representative: **Mewburn Ellis LLP**
**City Tower**
**40 Basinghall Street**
**London EC2V 5DE (GB)**

(56) References cited:
**EP-A1- 2 143 708**
**EP-A1- 2 355 197**
**WO-A1-2005/107335**
**WO-A1-2008/032617**
**WO-A1-2010/058776**
**WO-A1-2010/058777**
**WO-A1-2010/058777**
**JP-A- 2011 023 711**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a charge-transporting varnish. More specifically, the invention relates to a charge-transporting varnish which includes a charge-transporting material composed of an aniline derivative and a dopant material composed of phosphotungstic acid.

### BACKGROUND ART

A charge-transporting thin-film composed of an organic compound is used as the emissive layer or charge injection layer in organic electroluminescence (abbreviated below as "organic EL") devices.

Processes for forming such a charge-transporting thin-film are broadly divided into dry processes such as vapor deposition and wet processes such as spin coating. Comparing dry processes and wet processes, the latter are capable of efficiently producing thin-films having a high flatness over a large surface area. Here, in the organic EL field where thin-films of larger surface area are desired, thin-films are often formed by wet processes.

In light of the above, the inventors have developed charge-transporting varnishes for the production, by wet processes, of charge-transporting thin-films applicable to various types of electronic devices (see, for example, Patent Document 1). See also Patent Document 2 which also recites charge-transporting varnish, a charge-transporting thin-film produced therefrom, and organic an electroluminescent device comprising the charge-transporting thin film.

However, in the field of organic EL devices recently, to efficiently manufacture devices in a high yield, even in the case of film formation by wet processes, there exists a growing desire for varnishes which can be baked in an inert gas atmosphere or at reduced pressure.

Also, given the trend today toward lighter and thinner electronic devices, substrates composed of organic compounds have come to be used in place of glass substrates. Consequently, varnishes which can be baked at a lower temperature than previously and moreover which, even in such cases, provide thin-films endowed with good charge transportability have also been desired. However, existing varnishes are sometimes unable to fully satisfy such needs.

### PRIOR ART DOCUMENT

### PATENT DOCUMENT

Patent Document 1: JP-A 2002-151272 Patent Document 2: WO 2010-058777

### SUMMARY OF THE INVENTION

### PROBLEMS TO BE SOLVED BY THE INVENTION

It is therefore an object of the present invention to provide a charge-transporting varnish which can be baked at a low temperature and under conditions where sufficient oxygen is not present, such as in an inert gas atmosphere or at reduced pressure, and which yields a thin-film produced under such baking conditions that has a high flatness and a high charge transportability and that, when applied in organic EL devices, is able to elicit excellent EL device characteristics.

### MEANS FOR SOLVING THE PROBLEMS

The inventors have conducted extensive investigations, as a result of which they have discovered that, in a varnish which includes a charge-transporting material composed of an aniline derivative, a dopant material composed of phosphotungstic acid, and an organic solvent, by adjusting within a given range the ratio $W_D/W_H$ of the mass $W_D$ of phosphotungstic acid included in the varnish to the mass $W_H$ of charge-transporting material, the varnish can be baked at a low temperature and under conditions where sufficient oxygen is not present, such as in an inert gas atmosphere or at reduced pressure, that the thin-film produced under such baking conditions has a high flatness and a high charge transportability, and moreover that when such a thin-film is employed as a hole injection layer, an organic EL device capable of exhibiting excellent brightness characteristics can be obtained.

Accordingly, the invention provides:

1. A charge-transporting varnish characterized by including a charge-transporting material composed of an aniline derivative of formula (1)

[Chemical Formula 1]

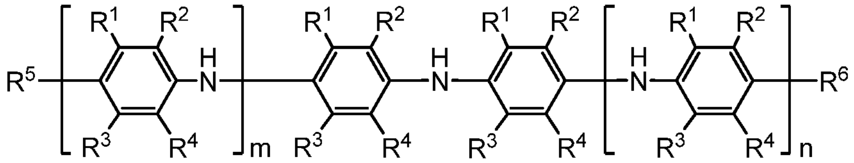

(1)

wherein $R^1$ to $R^6$ are each independently a hydrogen atom, a halogen atom, a nitro group, a cyano group, an amino group, an alkyl group of 1 to 20 carbons, an alkenyl group of 2 to 20 carbons or an alkynyl group of 2 to 20 carbons which may be substituted with $Z^1$, an aryl group of 6 to 20 carbons or a heteroaryl group of 2 to 20 carbons which may be substituted with $Z^2$, $-NHY^1$, $-NY^2Y^3$, $-OY^4$ or $-SY^5$; $Y^1$ to $Y^5$ are each independently an alkyl group of 1 to 20 carbons, an alkenyl group of 2 to 20 carbons or an alkynyl group of 2 to 20 carbons which may be substituted with $Z^1$, or an aryl group of 6 to 20 carbons or a heteroaryl group of 2 to 20 carbons which may be substituted with $Z^2$; $Z^1$ is a halogen atom, a nitro group, a cyano group, an amino group, or an aryl group of 6 to 20 carbons or a heteroaryl group of 2 to 20 carbons which may be substituted with $Z^3$; $Z^2$ is a halogen atom, a nitro group, a cyano group, an amino group, or an alkyl group of 1 to 20 carbons, an alkenyl group of 2 to 20 carbons or an alkynyl group of 2 to 20 carbons which may be substituted with $Z^3$; and $Z^3$ is a halogen atom, a nitro group, a cyano group or an amino group; and m and n are each independently an integer from 1 to 5, a dopant material consisting of phosphotungstic acid, and an organic solvent, wherein the ratio $W_D/W_H$ of the mass $W_D$ of the dopant material to the mass $W_H$ of the charge-transporting material satisfies the relationship $3.5 \leq W_D/W_H \leq 15.0$; and wherein the molecular weight of the aniline derivative is from 300 to 5,000.

2. The charge-transporting varnish according to 1 above, wherein $R^1$ to $R^6$ are each independently a hydrogen atom, a halogen atom, an alkyl group of 1 to 20 carbons which may be substituted with $Z^1$, an aryl group of 6 to 20 carbons which may be substituted with $Z^2$, $-NHY^1$, $-NY^2Y^3$, $OY^4$ or $-SY^5$; $Y^1$ to $Y^5$ are each independently an alkyl group of 1 to 10 carbons which may be substituted with $Z^1$ or an aryl group of 6 to 10 carbons which may be substituted with $Z^2$; $Z^1$ is a halogen atom or an aryl group of 6 to 10 carbons which may be substituted with $Z^3$; and $Z^2$ is a halogen atom or an alkyl group of 1 to 10 carbons which may be substituted with $Z^3$;

3. A charge-transporting thin-film produced using the charge-transporting varnish according to 1 or 2 above;

4. A charge-transporting thin-film produced using a charge-transporting material composed of an aniline derivative of formula (1)

[Chemical Formula 2]

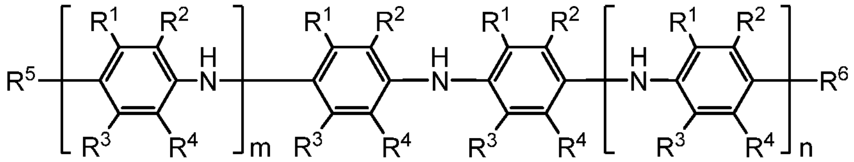

(1)

wherein $R^1$ to $R^6$ are each independently a hydrogen atom, a halogen atom, a nitro group, a cyano group, an amino group, an alkyl group of 1 to 20 carbons, an alkenyl group of 2 to 20 carbons or an alkynyl group of 2 to 20 carbons which may be substituted with $Z^1$, an aryl group of 6 to 20 carbons or a heteroaryl group of 2 to 20 carbons which may be substituted with $Z^2$, $-NHY^1$, $-NY^2Y^3$, $-OY^4$ or $-SY^5$; $Y^1$ to $Y^5$ are each independently an alkyl group of 1 to 20 carbons, an alkenyl group of 2 to 20 carbons or an alkynyl group of 2 to 20 carbons which may be substituted with $Z^1$, or an aryl group of 6 to 20 carbons or a heteroaryl group of 2 to 20 carbons which may be substituted with $Z^2$; $Z^1$ is a halogen atom, a nitro group, a cyano group, an amino group, or an aryl group of 6 to 20 carbons or a heteroaryl group of 2 to 20 carbons which may be substituted with $Z^3$; $Z^2$ is a halogen atom, a nitro group, a cyano group, an amino group, or an alkyl group of 1 to 20 carbons, an alkenyl group of 2 to 20 carbons or an alkynyl group of 2 to 20 carbons which may be substituted with $Z^3$; and $Z^3$ is a halogen atom, a nitro group, a cyano group or an amino group; and m and n are each independently an integer from 1 to 5) and a dopant material consisting of phosphotungstic acid, wherein the ratio $W_D/W_H$ of the mass $W_D$ of the dopant material to the mass $W_H$ of the charge-transporting material satisfies the relationship $3.5 \leq W_D/W_H \leq 15.0$; and wherein the molecular weight of the aniline derivative is from 300 to 5,000.

5. An electronic device having the charge-transporting thin-film of 3 or 4 above;

6. An organic electroluminescence device having the charge-transporting thin-film of 3 or 4 above;
7. The organic electroluminescence device according to 6 above, wherein the charge-transporting thin-film is a hole injection layer or a hole transport layer;
8. A method of producing a charge-transporting thin-film, characterized by applying the charge-transporting varnish of 1 or 2 above onto a substrate and evaporating off the solvent;
9. The method of producing a charge-transporting thin-film according to 8 above, wherein the solvent is evaporated off by baking the applied varnish in a nitrogen atmosphere;
10. The method of producing a charge-transporting thin-film according to 8 above, wherein the solvent is evaporated off by baking the applied varnish in a vacuum;
11. A method of producing an organic electroluminescence device using the charge-transporting thin-film of 3 or 4 above; and
12. A method of producing an organic electroluminescence device using the charge-transporting thin-film obtained by the production method of any one of 8 to 10 above.

## ADVANTAGEOUS EFFECTS OF THE INVENTION

The charge-transporting varnish of the invention, because it includes both a charge-transporting material composed of an aniline derivative and also phosphotungstic acid, and because the ratio $W_D/W_H$ of the mass $W_D$ of the phosphotungstic acid to the mass $W_H$ of the charge-transporting material has been adjusted within a given range, can be baked at a low temperature and under conditions where sufficient oxygen is not present, such as in an inert gas atmosphere or at reduced pressure. Moreover, a thin-film produced under such baking conditions has a high flatness and a high charge transportability. In cases where such a thin-film is employed as a hole injection layer, an organic EL device capable of exhibiting excellent brightness characteristics can be obtained. Hence, by using the charge-transporting varnish of this invention, it is possible to achieve higher efficiency and higher yield in the production of such devices owing in part to the greater simplicity of the production process, or to render such devices more lightweight and compact.
Also, the charge-transporting varnish of the invention can reproducibly produce thin-films of excellent charge transportability even using various wet processes capable of film formation over a large surface area, such as a spin coating process or a slit coating process, and is thus capable of fully accommodating recent advances in the field of organic EL devices.
In addition, thin-films obtained from the inventive charge-transporting varnish can also be used as, for example, antistatic coatings or as anode buffer layers in organic thin-film solar cells.

## EMBODIMENT FOR CARRYING OUT THE INVENTION

The invention is described more fully below.
The charge-transporting varnish according to this invention includes a charge-transporting material composed of an aniline derivative of formula (1), a dopant material composed of phosphotungstic acid, and an organic solvent, wherein the ratio $W_D/W_H$ of the mass $W_D$ of the dopant material to the mass $W_H$ of the charge-transporting material satisfies the relationship $3.5 \leq W_D/W_H \leq 15.0$.
Here, "charge-transporting" is synonymous with electrically conductive, and means the same thing as hole-transporting. The charge-transporting varnish of the invention may itself have charge transportability, or the solid film obtained using the varnish may have charge transportability.

[Chemical Formula 3]

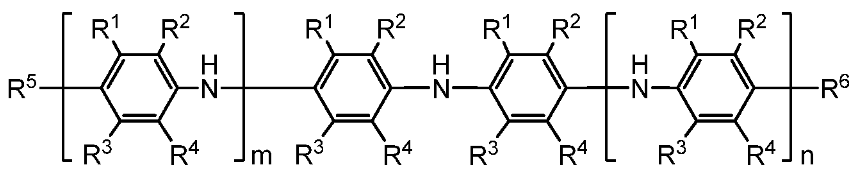

(1)

In formula (1), $R^1$ to $R^6$ are each independently a hydrogen atom, a halogen atom, a nitro group, a cyano group, an amino group, an alkyl group of 1 to 20 carbons which may be substituted with $Z^1$, an alkenyl group of 2 to 20 carbons which may be substituted with $Z^1$, an alkynyl group of 2 to 20 carbons which may be substituted with $Z^1$, an aryl group of 6 to 20 carbons which may be substituted with $Z^2$, a heteroaryl group of 2 to 20 carbons which may be substituted with $Z^2$, $-NHY^1$, $-NY^2Y^3$, $-OY^4$ or $-SY^5$. Here, $Z^1$ is a halogen atom, a nitro group, a cyano group, an amino group, an

aryl group of 6 to 20 carbons which may be substituted with $Z^3$ or a heteroaryl group of 2 to 20 carbons which may be substituted with $Z^3$; $Z^2$ is a halogen atom, a nitro group, a cyano group, an amino group, an alkyl group of 1 to 20 carbons which may be substituted with $Z^3$, an alkenyl group of 2 to 20 carbons which may be substituted with $Z^3$ or an alkynyl group of 2 to 20 carbons which may be substituted with $Z^3$; and $Z^3$ is a halogen atom, a nitro group, a cyano group or an amino group.

Illustrative examples of the halogen atom include fluorine, chlorine, bromine and iodine atoms.

The alkyl group of 1 to 20 carbons may be linear, branched or cyclic. Illustrative examples include linear or branched alkyl groups of 1 to 20 carbons such as methyl, ethyl, n-propyl, isopropyl, n-butyl, isobutyl, s-butyl, t-butyl, n-pentyl, n-hexyl, n-heptyl, n-octyl, n-nonyl and n-decyl; and cyclic alkyl groups of 3 to 20 carbons such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl.

Illustrative examples of alkenyl groups of 2 to 20 carbons include ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pentenyl, n-1-decenyl and n-1-eicosenyl.

Illustrative examples of alkynyl groups of 2 to 20 carbons include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl.

Illustrative examples of aryl groups of 6 to 20 carbons include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl.

Illustrative examples of heteroaryl groups of 2 to 20 carbons include 2-thienyl, 3-thienyl, 2-furanyl, 3-furanyl, 2-oxazoylyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl.

$-NHY^1$ is a moiety in which one hydrogen atom on an amino group ($-NH_2$) is substituted with $Y^1$, $NY^2Y^3$ is a moiety in which the hydrogen atoms on an amino group ($-NH_2$) are substituted with $Y^2$ and $Y^3$, $-OY^4$ is a moiety in which the hydrogen atom on a hydroxyl group (-OH) is substituted with $Y^4$, and $-SY^5$ is a moiety in which the hydrogen atom on a thiol group (-SH) is substituted with $Y^5$.

$Y^1$ to $Y^5$ are each independently an alkyl group of 1 to 20 carbons which may be substituted with $Z^1$, an alkenyl group of 2 to 20 carbons which may be substituted with $Z^1$, an alkynyl group of 2 to 20 carbons which may be substituted with $Z^1$, an aryl group of 6 to 20 carbons which may be substituted with $Z^2$ or a heteroaryl group of 2 to 20 carbons which may be substituted with $Z^2$. These alkyl groups, alkenyl groups, alkynyl groups, aryl groups and heteroaryl groups are exemplified by the same groups as mentioned above.

Of these, $R^1$ to $R^6$ are preferably hydrogen atoms, halogen atoms, alkyl groups of 1 to 20 carbons which may be substituted with $Z^1$, aryl groups of 6 to 20 carbons which may be substituted with $Z^2$, $-NHY^1$, $-NY^2Y^3$, $-OY^4$ or $-SY^5$. In this case, $Y^1$ to $Y^5$ are preferably alkyl groups of 1 to 10 carbons which may be substituted with $Z^1$ or aryl groups of 6 to 10 carbons which may be substituted with $Z^2$, more preferably alkyl groups of 1 to 6 carbons which may be substituted with $Z^1$ or phenyl groups which may be substituted with $Z^2$, and even more preferably alkyl groups of 1 to 6 carbons or phenyl groups.

In particular, $R^1$ to $R^6$ are more preferably hydrogen atoms, fluorine atoms, methyl groups, phenyl groups or diphenylamino groups ($-NY^2Y^3$, wherein $Y^2$ and $Y^3$ are phenyl groups), especially $R^1$ to $R^4$ are even more preferably hydrogen atoms and both $R^5$ and $R^6$ are at the same time more preferably hydrogen atoms or diphenylamino groups.

The alkyl groups, alkenyl groups and alkynyl groups of $R^1$ to $R^6$ and $Y^1$ to $Y^5$ may be substituted with $Z^1$, which is a halogen atom, nitro group, cyano group, amino group, aryl group of 6 to 20 carbons which may be substituted with $Z^3$ or a heteroaryl group of 2 to 20 carbons which may be substituted with $Z^3$. The aryl groups and heteroaryl groups of $R^1$ to $R^6$ and $Y^1$ to $Y^5$ may be substituted with $Z^2$, which is a halogen atom, nitro group, cyano group, amino group, alkyl group of 1 to 20 carbons which may be substituted with $Z^3$, alkenyl group of 2 to 20 carbons which may be substituted with $Z^3$ or alkynyl group of 2 to 20 carbons which may be substituted with $Z^3$.

Such alkyl groups, alkenyl group, alkynyl groups, aryl groups and heteroaryl groups are exemplified by the same groups as mentioned above. These groups may be additionally substituted with $Z^3$, which is a halogen atom, nitro group, cyano group or amino group (the halogen atom being exemplified in the same way as above).

In particular, in $R^1$ to $R^6$ and $Y^1$ to $Y^5$, $Z^1$ is preferably a halogen atom or an aryl group of 6 to 10 carbons which may be substituted with $Z^3$, more preferably a fluorine atom or a phenyl group, and even more preferably does not exist (i.e., does not substitute $R^1$ to $R^6$ and $Y^1$ to $Y^5$) ; and $Z^2$ is preferably a halogen atom or an alkyl group of 1 to 10 carbons which may be substituted with $Z^3$, more preferably a fluorine atom or an alkyl group of 1 to 6 carbons, and even more preferably does not exist (i.e., does not substitute $R^1$ to $R^6$ and $Y^1$ to $Y^5$).

In addition, $Z^3$ is preferably a halogen atom, more preferably a fluorine atom, and even more preferably does not exist (i.e., does not substitute $R^1$ to $R^6$ and $Y^1$ to $Y^5$).

The letters "m" and "n" are each independently integers from 1 to 5. From the standpoint of increasing the

solubility of the aniline derivative, it is preferable for m+n ≤ 8, and more preferable for m+n ≤ 5.

The molecular weight of the aniline derivative used in this invention is from 300 to 5,000. From the standpoint of increasing the solubility thereof, the molecular weight is preferably 3,000 or less, and more preferably 2,000 or less.

Examples of preferred aniline derivatives in this invention are shown below, although the invention is not limited to these alone.

[Chemical Formula 4]

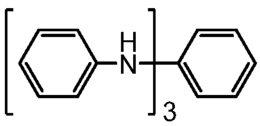

(a)

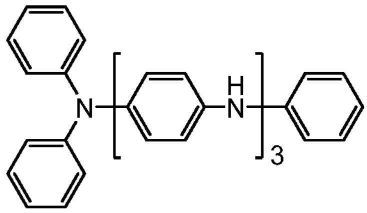

(b)

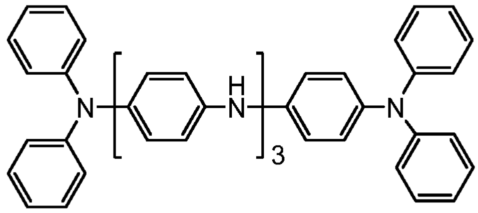

(c)

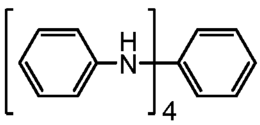

(d)

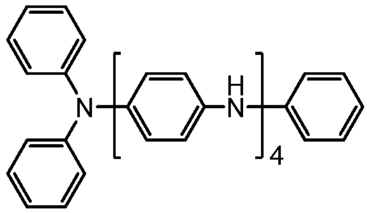

(e)

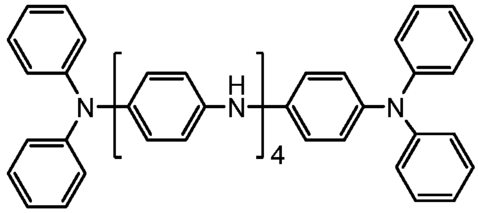

(f)

As mentioned above, the charge-transporting varnish of the invention includes phosphotungstic acid, which is one type of heteropolyacid compound.

A heteropolyacid compound is a polyacid having a structure in which a heteroatom is positioned at the center of the molecule--typically of the Keggin-type chemical structure shown in formula (A) or the Dawson-type chemical structure shown in formula (B), and which is obtained by the condensation of an isopolyacid which is an oxo acid of vanadium (V), molybdenum (Mo), tungsten (W) or the like with an oxo acid of a different element. Examples of this oxo acid of another element include primarily oxo acids of silicon (Si), phosphorus (P) and arsenic (As).

[Chemical Formula 5]

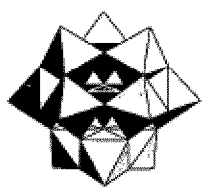

(A)

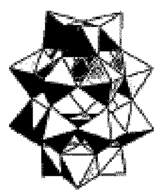

(B)

Phosphotungstic acid (typically referred to as "phosphotungstic acid hydrate" or "12-tungstophosphoric acid n-hydrate") is available as a commercial product. Alternatively phosphotungstic acid may be synthesized by a known method.

Phosphotungstic acid is generally represented by the chemical formula $H_3 (PW_{12}O_{40}) \cdot nH_2O$. However, even phosphotungstic acid for which, in quantitative analysis such as elemental analysis, the number of phosphorus, oxygen or tungsten atoms in this formula is high or low may be used in this invention, provided it was acquired as a commercial product or suitably synthesized in accordance with a known method of synthesis. In such cases, the mass of the phosphotungstic acid specified in this invention refers not to the mass of pure phosphotungstic acid (phosphotungstic acid content) within the product of synthesis or the commercial product, but rather, in the form available as a commercial product or the form that can be isolated by a known method of synthesis, to the total mass in a state that includes water of hydration and other impurities.

In the charge-transporting varnish of the invention, it is essential for the charge-transporting material and the phosphotungstic acid (dopant material) to be present in a mass ratio of phosphotungstic acid to charge-transporting material of from 3.5:1 to 15.0:1.

That is, the ratio of the mass of phosphotungstic acid ($W_D$) to the mass of charge-transporting material ($W_H$) must satisfy the relationship $3.5 \leq W_D/W_H \leq 15.0$, preferably satisfies the relationship $3.6 \leq W_D/W_H \leq 14.0$, more preferably satisfies the relationship $3.7 \leq W_D/W_H \leq 13.0$, even more preferably satisfies the relationship $3.8 \leq W_D/W_H \leq 12.0$, and still more preferably satisfies, in increasing degrees of preference: $3.9 \leq W_D/W_H \leq 11.0$, $4.0 \leq W_D/W_H \leq 10.0$, $4.5 \leq W_D/W_H \leq 9.0$ and $5.0 \leq W_D/W_H \leq 7.0$.

At $W_D/W_H$ below 3.5 or greater than 15.0, a thin-film having excellent charge transportability cannot be obtained when the varnish is baked at a low temperature and under conditions where sufficient oxygen is not present, such as in an inert gas atmosphere or in a vacuum, and so the brightness characteristics of organic EL devices in which this thin-film is used cannot be enhanced.

In this invention, "in a vacuum" refers to a pressure lower than atmospheric pressure ($1.013\times10^5$ Pa) and is synonymous with "under a reduced pressure."

The solvent used when preparing the charge-transporting varnish may be a high-solvency solvent which is capable of dissolving well the charge-transporting material and the dopant material. Illustrative examples of such high-solvency solvents that may be used include organic solvents such as N,N-dimethylformamide, N,N-dimethylacetamide, N-methylpyrrolidone, 1,3-dimethyl-2-imidazolidinone and diethylene glycol monomethyl ether. These solvents may be used singly or two or more may be used in admixture. The amount of use may be set to from 5 to 100 wt % of the overall solvent used in the varnish.

It is preferable for both the charge-transporting material and the dopant material to be either completely dissolved or in a uniformly dispersed state, within the solvent.

In addition, at least one high-viscosity organic solvent having a viscosity at 25°C of from 10 to 200 mPa·s, and especially 35 to 150 mPa·s, and having a boiling point at standard pressure (atmospheric pressure) of from 50 to 300°C, and especially from 150 to 250°C, may be included in the charge-transporting varnish of the invention. By adding such a solvent, adjusting the viscosity of the varnish is easy, thin-films of high flatness are reproducibly provided, and varnish preparation in accordance with the coating method to be used is easy.

Illustrative examples of high-viscosity organic solvents include, but are not particularly limited to, cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol and hexylene glycol. These solvents may be used singly, or two or more may be used in admixture.

The amount of high-viscosity organic solvent added as a proportion of the overall solvent used in the varnish of the invention is preferably within a range where no precipitation of solids occurs. To the extent that solids do not precipitate, the amount of such addition is preferably from 5 to 80 wt %.

In addition, another solvent may be admixed in a proportion with respect to the overall solvent used in the varnish of from 1 to 90 wt %, and preferably 1 to 50 wt %, for such purposes as to enhance the substrate wettability, adjust the solvent surface tension, adjust the solvent polarity and adjust the solvent boiling point.

Examples of such solvents include, but are not limited to, ethylene glycol monobutyl ether, diethylene glycol diethyl ether, diethylene glycol dimethyl ether, diethylene glycol monoethyl ether acetate, diethylene glycol monobutyl ether acetate, dipropylene glycol monomethyl ether, propylene glycol monomethyl ether, propylene glycol monomethyl ether acetate, diethylene glycol monoethyl ether, diacetone alcohol, γ-butyrolactone, ethyl lactate and n-hexyl acetate. These solvents may be used singly or two or more may be used in admixture.

The viscosity of the inventive varnish is suitably set according to such factors as the thickness of the thin-film to be produced and the solids concentration of the varnish, and is generally from 1 to 50 mPa·s at 25°C.

The solids concentration of the charge-transporting varnish in this invention is suitably set while taking into consideration the viscosity, surface tension and other properties of the varnish and the thickness and other properties of the thin-film to be produced, and is generally from about 0.1 wt % to about 10.0 wt %. Taking into account such considerations as improving the varnish coatability and minimizing the settling of solids, the solids concentration of the varnish is preferably from 0.5 to 5.0 wt %, and more preferably from 1.0 to 3.0 wt %.

By coating the above-described charge-transporting varnish onto a substrate and evaporating off the solvent, a charge-transporting thin-film can be formed on the substrate.

Examples of the varnish coating method include, but are not particularly limited to, dipping, spin coating, transfer printing, roll coating, brush coating, inkjet printing and spraying. It is preferable to adjust the viscosity and surface tension of the varnish according to the coating method to be used.

When using the varnish of the invention, the solvent evaporation (baking) atmosphere is not particularly limited. A thin-film having a uniform film surface and high charge transportability can be obtained not only in an open-air atmosphere, but even in an inert gas such as nitrogen or in a vacuum.

The baking temperature is not particularly limited, provided the solvent can be evaporated off, although a baking

temperature of from 40 to 250°C is preferred. From the standpoint of reproducibly obtaining a thin-film having a high charge transportability, the baking temperature is more preferably at least 100°C, even more preferably at least 130°C, and still more preferably at least 140°C. In this case, a temperature change in two or more steps may be applied for such purposes as to manifest a more uniform film formability or to induce the reaction to proceed on the substrate. Heating may be carried out using a suitable apparatus such as a hot plate or an oven.

The thickness of the charge-transporting thin-film is not particularly limited; when used as a hole injection layer in an organic EL device, a film thickness of from 5 to 200 nm is preferred. Methods of varying the film thickness include varying the solids concentration in the varnish, and varying the amount of solution on the substrate during coating.

Examples of the materials and method used to fabricate organic light-emitting diodes (OLEDs) using the charge-transporting varnish of the invention include, but are not limited to, those mentioned below.

The electrode substrate to be used is preferably cleaned by carrying out liquid washing beforehand with a cleaning agent, alcohol, pure water or the like. For example, in the case of a positive electrode substrate, it is preferable to carry out surface treatment such as UV/ozone treatment or oxygen-plasma treatment just prior to use. However, in cases where the positive electrode material is composed primarily of an organic substance, surface treatment need not be carried out.

An example of a method of manufacturing an OLED having a hole injection layer composed of a thin-film obtained from the charge-transporting varnish of the invention is described below.

A hole injection layer is formed on an electrode by coating the charge-transporting varnish of the invention onto a positive electrode substrate, then carrying out evaporation and baking by the above method. The thus obtained substrate is introduced into a vacuum deposition system and a hole transport layer, emissive layer, electron transport layer, electron injection layer and negative electrode metal are vapor-deposited in this order to form the OLED. Carrier block layers may be provided to desired layer intervals so as to control the light-emitting region.

Illustrative examples of the positive electrode material include transparent electrodes such as indium-tin oxide (ITO) and indium-zinc oxide (IZO). A positive electrode material on which planarizing treatment has been carried out is preferred. Use can also be made of polythiophene derivatives and polyaniline derivatives having a high charge transportability.

Illustrative examples of the hole transport layer-forming material include triarylamines such as (triphenylamine) dimer (TPD) derivatives, N,N'-di(l-naphthyl)-N,N'-diphenylbenzidine ($\alpha$-NPD) and [(triphenylamine) dimer] spirodimer (Spiro-TAD); starburst amines such as 4,4',4"-tris[3-methylphenyl(phenyl)amino]triphenylamine (m-MTDATA) and 4,4',4"-tris[1-naphthyl(phenyl)amino]triphenylamine (1-TNATA); and oligothiophenes such as 5,5"-bis-{4-[bis(4-methylphenyl)amino]phenyl}-2,2':5',2"-terthiophene (BMA-3T).

Illustrative examples of the emissive layer-forming material include tris(8-quinolinolate) aluminum(III) ($Alq_3$), bis(8-quinolinolate) zinc(II) ($Znq_2$), bis(2-methyl-8-quinolinolate)(p-phenylphenolate) aluminum(III) (BAlq) and 4,4'-bis(2,2-diphenylvinyl)biphenyl (DPVBi). It is also possible to form the emissive layer by the co-vapor deposition of an electron-transporting material or hole-transporting material with a light-emitting dopant.

Illustrative examples of the electron-transporting material include $Alq_3$, BAlq, DPVBi, (2-(4-biphenyl)-5-(4-t-butylphenyl)-1,3,4-oxadiazole) (PBD), triazole derivatives (TAZ), bathocuproin (BCP) and silole derivatives.

Illustrative examples of light-emitting dopants include quinacridone, rubrene, coumarin 540, 4-(dicyanomethylene)-2-methyl-6-(p-dimethylaminostyryl)-4H-pyran (DCM), tris(2-phenylpyridine) iridium(III) ($Ir(ppy)_3$) and (1,10-phenanthroline)tris(4,4,4-trifluoro-1-(2-thienyl) butane-1,3-dionate) europium(III) ($Eu(TTA)_3phen$).

Illustrative examples of the carrier block layer-forming material include PBD, TAZ and BCP.

Illustrative examples of the electron injection layer-forming material include lithium oxide ($Li_2O$), magnesium oxide (MgO) , alumina ($Al_2O_3$), lithium fluoride (LiF), magnesium fluoride ($MgF_2$), strontium fluoride ($SrF_2$), Liq, Li(acac), lithium acetate and lithium benzoate.

Illustrative examples of the negative electrode material include aluminum, magnesium-silver alloys, aluminum-lithium alloys, lithium, sodium, potassium and cesium.

The method of fabricating polymer LEDs (PLEDs) using the charge-transporting varnish of the invention, although not particularly limited, is exemplified by the following method.

A PLED having a hole injection layer composed of a thin-film obtained from the charge-transporting varnish of the invention can be fabricated by, in the fabrication of an OLED as described above, successively forming a hole-transporting polymer layer and a light-emitting polymer layer instead of carrying out vacuum deposition operations for a hole transport layer, emissive layer, electron transport layer and electron injection layer.

Specifically, the charge-transporting varnish of the invention is applied onto a positive electrode substrate and a hole injection layer is produced by the above method. A hole-transporting polymer layer and a light-emitting polymer layer are successively formed on top thereof, in addition to which a negative electrode material is vapor-deposited, thereby forming a PLED.

The negative electrode and positive electrode materials used here may be similar to those used when fabricating an OLED as described above, and similar cleaning treatment and surface treatment may be carried out.

The method of forming the hole-transporting polymer layer and the light-emitting polymer layer is exemplified by a film-forming method in which a hole-transporting polymer material or a light-emitting polymer material, or a material obtained by adding thereto a dopant material, is dissolved or uniformly dispersed by the addition of a solvent, following which the resulting solution or dispersion is coated onto the hole injection layer or the hole-transporting polymer layer, and the solvent is subsequently removed by evaporation.

Illustrative examples of the hole-transporting polymer material include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)-benzidine] end-capped with polysilsesquioxane and poly[(9,9-didioctylfluorenyl-2,7-diyl)-co-(4,4'-(N-(p-butylphenyl))diphenylamine)].

Illustrative examples of light-emitting polymer materials include polyfluorene derivatives such as poly(9,9-dialkylfluorene) (PDAF), polyphenylenevinylene derivatives such as poly(2-methoxy-5-(2'-ethylhexoxy)-1,4-phenylenevinylene) (MEH-PPV), polythiophene derivatives such as poly(3-alkylthiophene) (PAT), and polyvinylcarbazole (PVCz).

Illustrative examples of the solvent include toluene, xylene and chloroform. Examples of the method of dissolution or uniform dispersion include stirring, stirring under applied heat, and ultrasonic dispersion.

Examples of the coating method include, but are not particularly limited to, inkjet printing, spraying, dipping, spin coating, transfer printing, roll coating and brush coating. Coating is preferably carried out in an inert gas atmosphere such as nitrogen or argon.

Examples of the solvent evaporation method include methods that involve heating in an oven or on a hot plate and within an inert gas atmosphere or in a vacuum.

## EXAMPLES

Synthesis Examples, Working Examples and Comparative Examples are given below to more concretely illustrate the invention, although the invention is not limited by these Examples. The equipment used in the Working Examples and Comparative Examples was as follows.

(1) $^{1}$H-NMR Measurement:
Varian high-resolution NMR spectrometer

(2) Substrate Cleaning:
Substrate cleaning machine (reduced-pressure plasma system), from Choshu Industry Co., Ltd.

(3) Varnish Coating:
MS-A100 Spin Coater, from Mikasa Co., Ltd.

(4) Film Thickness Measurement:
Surfcorder ET-4000 microfigure measuring instrument, from Kosaka Laboratory, Ltd.

(5) EL Device Fabrication:
C-E2L1G1-N Multifunction Vapor Deposition System, from Choshu Industry Co., Ltd.

(6) Measurement of EL Device Brightness, etc.:
I-V-L Measurement System from Tech World, Inc.

(7) EL Device Lifetime Measurement:
PEL-105S Organic EL Brightness Life Evaluation System, from EHC K.K.

[1] Synthesis of Charge-Transporting Material [Synthesis Example 1] Synthesis of Aniline Derivative

[Chemical Formula 6]

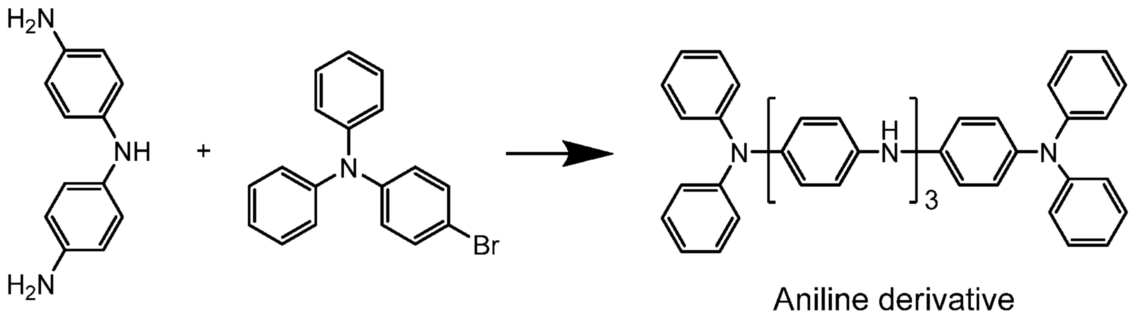


Tetrakis(triphenylphosphine)palladium, 0.5799 g (0.5018 mmol), and 10.13 g (105.40 mmol) of sodium tert-butoxide were added to a mixed suspension of 10.00 g (50.19 mmol) of 4,4'-diaminodiphenylamine, 34.17 g (105.40 mmol) of 4-bromotriphenylamine and 100 g of xylene, and the ingredients were stirred under nitrogen and at 130°C for 14 hours.

Next, the reaction mixture was subjected to filtration, a saturated saline solution was added to the filtrate, and liquid-liquid separation was carried out. The solid obtained by subsequently driving the solvent from the organic phase was recrystallized from 1,4-dioxane, giving the target aniline derivative in an amount of 22.37 g (yield, 65%).
$^1$H-NMR ($CDCl_3$) : δ7.83 (S, 2H), 7.68 (S, 1H), 7.26-7.20 (m, 8H), 7.01-6.89 (m, 28H).

[2] Preparation of Charge-Transporting Varnish

[Example 1-1]

An amount of 0.098 g of the aniline derivative obtained in Synthesis Example 1 and 0.392 g of phosphotungstic acid (Kanto Chemical Co., Ltd.) were dissolved in 8 g of 1,3-dimethyl-2-imidazolidinone (Wako Pure Chemical Industries, Ltd.) under a nitrogen atmosphere. Next, 12 g of cyclohexanol (Wako Pure Chemical Industries, Ltd.) and 4 g of propylene glycol (Kanto Chemical Co., Ltd.) were added to the resulting solution and stirring was carried out, thereby preparing a charge-transporting varnish (solids concentration, 2.0 wt %).

[Examples 1-2 to 1-5]

Aside from setting the amounts of aniline derivative and phosphotungstic acid used to, respectively, 0.082 g and 0.408 g, 0.070 g and 0.420 g, 0.054 g and 0.435 g, and 0.045 g and 0.445 g, charge-transporting varnishes were prepared by the same method as in Example 1-1.

[Comparative Examples 1 to 3]

Aside from setting the amounts of aniline derivative and phosphotungstic acid used to, respectively, 0.163 g and 0.327 g, 0.122 g and 0.367 g, and 0.023 g and 0.466 g, charge-transporting varnishes were prepared by the same method as in Example 1-1.

[Comparative Example 4]

Aside from setting the amount of aniline derivative used to 0.245 g and using 0.245 g of phosphomolybdic acid (Kanto Chemical Co., Ltd.) instead of 0.392 g of phosphotungstic acid, a charge-transporting varnish was prepared by the same method as in Example 1-1.

[Comparative Examples 5 to 9]

Aside from setting the amounts of aniline derivative and phosphomolybdic acid used to, respectively, 0.163 g and 0.327 g, 0.122 g and 0.367 g, 0.08 g and 0.392 g, 0.082 g and 0.408 g, and 0.070 g and 0.420 g, charge-transporting varnishes were prepared by the same method as in Comparative Example 4.

[3] Fabrication of Organic EL Devices and Evaluation of Their Characteristics

[Example 2-1]

The varnish obtained in Example 1-1 was coated onto an ITO substrate using a spin coater, then dried for 5 minutes at 50°C and baked in an open-air atmosphere at 230°C for 15 minutes, thereby forming a 30 nm uniform thin-film on the ITO substrate. A 25 mm × 25 mm × 0.7 mm (t) glass substrate with indium-tin oxide (ITO) patterned on the surface thereof to a film thickness of 150 nm was used as the ITO substrate. Prior to use, impurities on the surface were removed with an $O_2$ plasma cleaning system (150 W, 30 seconds).

Next, using a vapor deposition system (degree of vacuum, $1.0\times10^{-5}$ Pa), thin-films of N,N'-di(1-naphthyl)-N,N'-diphenylbenzidine (α-NPD), tris(8-quinolinolate)aluminum(III) ($Alq_3$), lithium fluoride and aluminum were successively deposited on the ITO substrate on which a thin-film had been formed, thereby giving an organic EL device. At this time, vapor deposition was carried out at a rate of 0.2 nm/s for α-NPD, $Alq_3$ and aluminum, and at a rate of 0.02 nm/s for lithium fluoride. The film thicknesses were set to, respectively, 30 nm, 40 nm, 0.5 nm and 120 nm.

To prevent the device characteristics from deteriorating due to the influence of oxygen, moisture and the like in air, the organic EL device was sealed with sealing substrates, following which the characteristics were evaluated. Sealing was carried out by the following procedure.

The organic EL device was placed between sealing substrates in a nitrogen atmosphere having an oxygen concentration of 2 ppm or less and a dew point of not more than -85°C, and the sealing substrates were laminated together using an adhesive (XNR5516Z-B1, from Nagase ChemteX Corporation). At this time, a desiccant (HD-071010W-40, from Dynic Corporation) was placed, together with the organic EL device, within the sealing substrates.

The laminated sealing substrates were irradiated with UV light (wavelength, 365 nm; dosage, 6,000 mJ/$cm^2$), then annealed at 80°C for 1 hour to cure the adhesive.

[Examples 2-2 to 2-5, and Comparative Examples 10 to 18]

Aside from using the varnishes obtained in, respectively, Examples 1-2 to 1-5 and Comparative Examples 1 to 9 instead of the varnish obtained in Example 1-1, EL devices were fabricated by the same method as in Example 2-1.

[Example 3-1]

Aside from setting the baking temperature to 160°C, an EL device was fabricated by the same method as in Example 2-1.

[Examples 3-2 to 3-5, and Comparative Examples 19 to 22]

Aside from using the varnishes obtained in, respectively, Examples 1-2 to 1-5 and Comparative Examples 1 to 3 and 5 instead of the varnish obtained in Example 1-1, EL devices were fabricated by the same method as in Example 3-1.

The current densities and brightnesses of the organic EL devices fabricated as described above were measured at a driving voltage of 5 V. The results are shown in Tables 1 and 2.

[Table 1]

| | Heteropolyacid | Baking temperature | Baking atmosphere | D/H (wt/wt) | Current density (mA/$cm^2$) | Brightness (cd/$m^2$) |
|---|---|---|---|---|---|---|
| Comparative Example 10 | phosphotungstic acid | 230°C | open air | 2 | 2 | 31 |
| Comparative Example 11 | phosphotungstic acid | 230°C | open air | 3 | 25 | 891 |
| Example 2-1 | phosphotungstic acid | 230°C | open air | 4 | 70 | 2,038 |
| Example 2-2 | phosphotungstic acid | 230°C | open air | 5 | 75 | 2,238 |
| Example 2-3 | phosphotungstic acid | 230°C | open air | 6 | 106 | 3,000 |

(continued)

| | Heteropolyacid | Baking temperature | Baking atmosphere | D/H (wt/wt) | Current density ($mA/cm^2$) | Brightness ($cd/m^2$) |
|---|---|---|---|---|---|---|
| Example 2-4 | phosphotungstic acid | 230°C | open air | 8 | 75 | 2,176 |
| Example 2-5 | phosphotungstic acid | 230°C | open air | 10 | 83 | 2,289 |
| Comparative Example 12 | phosphotungstic acid | 230°C | open air | 20 | 32 | 851 |
| Comparative Example 13 | phosphomolybdic acid | 230°C | open air | 1 | 4 | 27 |
| Comparative Example 14 | phosphomolybdic acid | 230°C | open air | 2 | 100 | 2,663 |
| Comparative Example 15 | phosphomolybdic acid | 230°C | open air | 3 | 50 | 1,272 |
| Comparative Example 16 | phosphomolybdic acid | 230°C | open air | 4 | 40 | 970 |
| Comparative Example 17 | phosphomolybdic acid | 230°C | open air | 5 | 16 | 306 |
| Comparative Example 18 | phosphomolybdic acid | 230°C | open air | 6 | 14 | 279 |

[Table 2]

| | Heteropolyacid | Baking temperature | Baking atmosphere | D/H (wt/wt) | Current density ($mA/cm^2$) | Brightness ($cd/m^2$) |
|---|---|---|---|---|---|---|
| Comparative Example 19 | phosphotungstic acid | 160°C | open air | 2 | 1 | 36 |
| Comparative Example 20 | phosphotungstic acid | 160°C | open air | 3 | 15 | 536 |
| Example 3-1 | phosphotungstic acid | 160°C | open air | 4 | 59 | 1,938 |
| Example 3-2 | phosphotungstic acid | 160°C | open air | 5 | 82 | 2,445 |
| Example 3-3 | phosphotungstic acid | 160°C | open air | 6 | 96 | 2,801 |
| Example 3-4 | phosphotungstic acid | 160°C | open air | 8 | 101 | 2,952 |
| Example 3-5 | phosphotungstic acid | 160°C | open air | 10 | 84 | 2,422 |
| Comparative Example 21 | phosphotungstic acid | 160°C | open air | 20 | 37 | 929 |
| Comparative Example 22 | phosphomolybdic acid | 160°C | open air | 2 | 49 | 1,288 |

As shown in Tables 1 and 2, in cases where a charge-transporting varnish containing phosphotungstic acid in a mass ratio range relative to the charge-transporting material of 3.5:1 to 15.0:1 (Examples 2-1 to 2-5, and 3-1 to 3-5)

was used, regardless of the baking temperature, it was possible to fabricate EL devices having excellent brightness characteristics.

On the other hand, when charge-transporting varnishes containing phosphotungstic acid outside of the above range (Comparative Examples 10 to 12 and 19 to 21) or charge-transporting varnishes containing phosphomolybdic acid (Comparative Examples 13 to 18 and 22) were used, both in high-temperature baking and in low-temperature baking, none of these varnishes furnished EL devices capable of exhibiting good brightness characteristics.

[Example 4-1]

Aside from setting the baking atmosphere to a nitrogen atmosphere, an EL device was fabricated by the same method as in Example 2-1.

[Examples 4-2 and 4-3, and Comparative Examples 23 to 25]

Aside from using the varnishes obtained in, respectively, Examples 1-2 and 1-3 and Comparative Examples 1, 2 and 5 instead of the varnish obtained in Example 1-1, EL devices were fabricated by the same method as in Example 4-1.

[Example 5-1]

Aside from setting the baking temperature to 200°C and setting the baking atmosphere to under reduced pressure (21.3 kPa), an EL device was fabricated by the same method as in Example 2-3.

The current densities and brightnesses of the organic EL devices fabricated as described above were measured at a driving voltage of 5 V. The results are shown in Table 3.

[Table 3]

| | Heteropolyacid | Baking temperature | Baking atmosphere | D/H (wt/wt) | Current density ($mA/cm^2$) | Brightness ($cd/m^2$) |
|---|---|---|---|---|---|---|
| Comparative Example 23 | phosphotungstic acid | 230°C | nitrogen | 2 | 7 | 180 |
| Comparative Example 24 | phosphotungstic acid | 230°C | nitrogen | 3 | 29 | 1,000 |
| Example 4-1 | phosphotungstic acid | 230°C | nitrogen | 4 | 63 | 1,972 |
| Example 4-2 | phosphotungstic acid | 230°C | nitrogen | 5 | 82 | 2,393 |
| Example 4-3 | phosphotungstic acid | 230°C | nitrogen | 6 | 100 | 2,923 |
| Example 5-1 | phosphotungstic acid | 200°C | reduced pressure | 6 | 69 | 2,142 |
| Comparative Example 25 | phosphomolybdic acid | 230°C | nitrogen | 2 | 8 | 143 |

As shown in Tables 1 and 3, in cases where a charge-transporting varnish containing phosphotungstic acid in a mass ratio range relative to the charge-transporting material of 3.5:1 to 15.0:1 (Examples 2-1 to 2-5, 4-1 to 4-3, and 5-1) was used, regardless of the baking atmosphere, it was possible to fabricate EL devices having excellent brightness characteristics.

On the other hand, in cases where a charge-transporting varnish containing phosphotungstic acid outside of the above range (Comparative Examples 23 and 24) or phosphomolybdic acid outside of the above range (Comparative Example 25) was used, whether in open-air baking or baking in an atmosphere where sufficient oxygen is not present (baking in a nitrogen atmosphere or baking under reduced pressure), none of these varnishes furnished EL devices capable of exhibiting good brightness characteristics.

Durability tests were carried out on the EL devices fabricated in Examples 2-1 to 2-4, 3-1 to 3-4, 4-1 to 4-3 and 5-1, and in Comparative Examples 15 and 22. Table 4 shows the brightness half-lives (initial brightness, 5,000 $cd/m^2$) .

[Table 4]

| | Half-life (h) |
|---|---|
| Example 2-1 | 454 |
| Example 2-2 | 417 |
| Example 2-3 | 422 |
| Example 2-4 | 361 |
| Example 3-1 | 436 |
| Example 3-2 | 393 |
| Example 3-3 | 474 |
| Example 3-4 | 341 |
| Example 4-1 | 415 |
| Example 4-2 | 421 |
| Example 4-3 | 399 |
| Example 5-1 | 457 |
| Comparative Example 15 | 283 |
| Comparative Example 22 | 172 |

As shown in Table 4, the brightness half-lives of the organic EL devices having charge-transporting thin-films produced in the examples of the invention are longer than those of the organic EL devices having charge-transporting thin-films produced in the comparative examples.

From these results, it is apparent that, by using phosphotungstic acid in a mass ratio range relative to the charge-transporting material of from 3.5:1 to 15.0:1, it is possible to produce charge-transporting thin-films --especially charge-transporting thin-films suitable as hole injection layers-- that enable organic EL devices having a high durability to be achieved.

## Claims

**1.** A charge-transporting varnish **characterized by** comprising a charge-transporting material composed of an aniline derivative of formula (1)

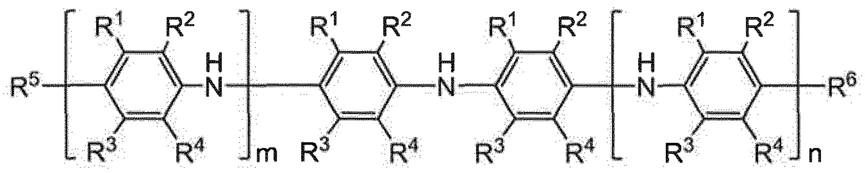 (1)

wherein $R^1$ to $R^6$ are each independently a hydrogen atom, a halogen atom, a nitro group, a cyano group, an amino group, an alkyl group of 1 to 20 carbons, an alkenyl group of 2 to 20 carbons or an alkynyl group of 2 to 20 carbons which may be substituted with $Z^1$, an aryl group of 6 to 20 carbons or a heteroaryl group of 2 to 20 carbons which may be substituted with $Z^2$, $-NHY^1$, $-NY^2Y^3$, $-OY^4$ or $-SY^5$;

$Y^1$ to $Y^5$ are each independently an alkyl group of 1 to 20 carbons, an alkenyl group of 2 to 20 carbons or an alkynyl group of 2 to 20 carbons which may be substituted with $Z^1$, or an aryl group of 6 to 20 carbons or a heteroaryl group of 2 to 20 carbons which may be substituted with $Z^2$;

$Z^1$ is a halogen atom, a nitro group, a cyano group, an amino group, or an aryl group of 6 to 20 carbons or a heteroaryl group of 2 to 20 carbons which may be substituted with $Z^3$;

$Z^2$ is a halogen atom, a nitro group, a cyano group, an amino group, or an alkyl group of 1 to 20 carbons, an alkenyl group of 2 to 20 carbons or an alkynyl group of 2 to 20 carbons which may be substituted with $Z^3$; and

$Z^3$ is a halogen atom, a nitro group, a cyano group or an amino group; and
m and n are each independently an integer from 1 to 5,
a dopant material consisting of phosphotungstic acid, and an organic solvent,
wherein the ratio $W_D/W_H$ of the mass $W_D$ of the dopant material to the mass $W_H$ of the charge-transporting material satisfies the relationship

$$3.5 \leq W_D/W_H \leq 15.0;$$

and
wherein the molecular weight of the aniline derivative is from 300 to 5,000.

2. The charge-transporting varnish according to claim 1, wherein $R^1$ to $R^6$ are each independently a hydrogen atom, a halogen atom, an alkyl group of 1 to 20 carbons which may be substituted with $Z^1$, an aryl group of 6 to 20 carbons which may be substituted with $Z^2$, $-NHY^1$, $-NY^2Y^3$, $OY^4$ or $-SY^5$;
$Y^1$ to $Y^5$ are each independently an alkyl group of 1 to 10 carbons which may be substituted with $Z^1$ or an aryl group of 6 to 10 carbons which may be substituted with $Z^2$;
$Z^1$ is a halogen atom or an aryl group of 6 to 10 carbons which may be substituted with $Z^3$; and
$Z^2$ is a halogen atom or an alkyl group of 1 to 10 carbons which may be substituted with $Z^3$.

3. A charge-transporting thin-film produced using the charge-transporting varnish according to claim 1 or 2.

4. A charge-transporting thin-film produced using a charge-transporting material composed of an aniline derivative of formula (1)

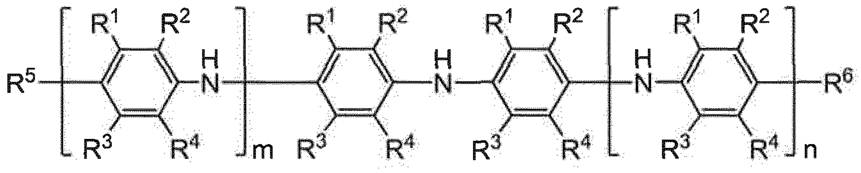

(1)

wherein $R^1$ to $R^6$ are each independently a hydrogen atom, a halogen atom, a nitro group, a cyano group, an amino group, an alkyl group of 1 to 20 carbons, an alkenyl group of 2 to 20 carbons or an alkynyl group of 2 to 20 carbons which may be substituted with $Z^1$, an aryl group of 6 to 20 carbons or a heteroaryl group of 2 to 20 carbons which may be substituted with $Z^2$, $-NHY^1$, $-NY^2Y^3$, $-OY^4$ or $-SY^5$;
$Y^1$ to $Y^5$ are each independently an alkyl group of 1 to 20 carbons, an alkenyl group of 2 to 20 carbons or an alkynyl group of 2 to 20 carbons which may be substituted with $Z^1$, or an aryl group of 6 to 20 carbons or a heteroaryl group of 2 to 20 carbons which may be substituted with $Z^2$;
$Z^1$ is a halogen atom, a nitro group, a cyano group, an amino group, or an aryl group of 6 to 20 carbons or a heteroaryl group of 2 to 20 carbons which may be substituted with $Z^3$;
$Z^2$ is a halogen atom, a nitro group, a cyano group, an amino group, or an alkyl group of 1 to 20 carbons, an alkenyl group of 2 to 20 carbons or an alkynyl group of 2 to 20 carbons which may be substituted with $Z^3$; and
$Z^3$ is a halogen atom, a nitro group, a cyano group or an amino group; and
m and n are each independently an integer from 1 to 5 and a dopant material consisting of phosphotungstic acid, wherein the ratio $W_D/W_H$ of the mass $W_D$ of the dopant material to the mass $W_H$ of the charge-transporting material satisfies the relationship

$$3.5 \leq W_D/W_H \leq 15.0;$$

and
wherein the molecular weight of the aniline derivative is from 300 to 5,000.

5. A charge-transporting varnish according to claim 1 or 2, or a charge-transporting thin-film according to claim 3 or 4, wherein the molecular weight of the aniline derivative is 3,000 or less.

6. An electronic device comprising the charge-transporting thin-film of claim 3 or 4.

**7.** An organic electroluminescence device comprising the charge-transporting thin-film of claim 3 or 4.

**8.** The organic electroluminescence device according to claim 7, wherein the charge-transporting thin-film is a hole injection layer or a hole transport layer.

**9.** A method of producing a charge-transporting thin-film, **characterized by** applying the charge-transporting varnish of claim 1 or 2 onto a substrate and evaporating off the solvent.

**10.** The method of producing a charge-transporting thin-film according to claim 9, wherein the solvent is evaporated off by baking the applied varnish in a nitrogen atmosphere.

**11.** The method of producing a charge-transporting thin-film according to claim 9, wherein the solvent is evaporated off by baking the applied varnish in a vacuum.

**12.** A method of producing an organic electroluminescence device using the charge-transporting thin-film of claim 3 or 4.

**13.** A method of producing an organic electroluminescence device using the charge-transporting thin-film obtained by the production method of any one of claims 9 to 11.

**Patentansprüche**

**1.** Ladungstransportlack, **dadurch gekennzeichnet, dass** dieser ein Ladungstransportmaterial, bestehend aus einem Anilinderivat der Formel (1):

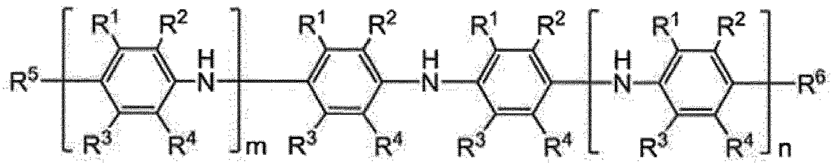 (1)

worin $R^1$ bis $R^6$ jeweils unabhängig ein Wasserstoffatom, ein Halogenatom, eine Nitrogruppe, eine Cyanogruppe, eine Aminogruppe, eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen, die gegebenenfalls mit $Z^1$ substituiert sind, eine Arylgruppe mit 6 bis 20 Kohlenstoffatomen oder eine Heteroarylgruppe mit 2 bis 20 Kohlenstoffatomen sind, die gegebenenfalls mit $Z^2$, $-NHY^1$, $-NY^2Y^3$, $-OY^4$ oder $-SY^5$ substituiert sind;
$Y^1$ bis $Y^5$ jeweils unabhängig eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen, die gegebenenfalls mit $Z^1$ substituiert sind, oder eine Arylgruppe mit 6 bis 20 Kohlenstoffatomen oder eine Heteroarylgruppe mit 2 bis 20 Kohlenstoffatomen sind, die gegebenenfalls mit $Z^2$ substituiert sind;
$Z^1$ ein Halogenatom, eine Nitrogruppe, eine Cyanogruppe, eine Aminogruppe oder oder eine Arylgruppe mit 6 bis 20 Kohlenstoffatomen oder eine Heteroarylgruppe mit 2 bis 20 Kohlenstoffatomen ist, die gegebenenfalls mit $Z^3$ substituiert sind;
$Z^2$ ein Halogenatom, eine Nitrogruppe, eine Cyanogruppe, eine Aminogruppe oder eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen ist, die gegebenenfalls mit $Z^3$ substituiert sind;
$Z^3$ in Halogenatom, eine Nitrogruppe, eine Cyanogruppe oder eine Aminogruppe ist; und
m und n jeweils unabhängig eine ganze Zahl von 1 bis 5 sind,
einem aus Wolframatophosphorsäure bestehenden Dotiermaterial und einem organischen Lösungsmittel besteht,
wobei das Verhältnis $W_D/W_H$ zwischen der Masse $W_D$ des Dotiermaterials und der Masse $W_H$ des Ladungstransportmaterials die folgende Beziehung erfüllt:

$$3{,}5 \leq W_D/W_H \leq 15{,}0;$$

und

wobei das Molekulargewicht des Anilinderivats 300 bis 5000 beträgt.

2. Ladungstransportlack nach Anspruch 1, worin $R^1$ bis $R^6$ jeweils unabhängig ein Wasserstoff, ein Halogenatom, eine Alkylgruppe mit 1 bis 20 Kohlenstoffatomen, die gegebenenfalls mit $Z^1$ substituiert ist, eine Arylgruppe mit 6 bis 20 Kohlenstoffatomen, die gegebenenfalls mit $Z^2$ substituiert ist, $-NHY^1$, $-NY^2Y^3$, $OY^4$ oder $-SY^5$ sind;
$Y^1$ bis $Y^5$ jeweils unabhängig eine Alkylgruppe mit 1 bis 10 Kohlenstoffatomen, die gegebenenfalls mit $Z^1$ substituiert ist, oder eine Arylgruppe mit 6 bis 10 Kohlenstoffatomen sind, die gegebenenfalls mit $Z^2$ substituiert ist;
$Z^1$ ein Halogenatom oder eine Arylgruppe mit 6 bis 10 Kohlenstoffatomen ist, die gegebenenfalls mit $Z^3$ substituiert ist;
$Z^2$ ein Halogenatom oder eine Alkylgruppe mit 1 bis 10 Kohlenstoffatomen ist, die gegebenenfalls mit $Z^3$ substituiert ist.

3. Ladungstransportdünnfilm, hergestellt unter Verwendung eines Ladungstransportlacks nach Anspruch 1 oder 2.

4. Ladungstransportdünnfilm, hergestellt unter Verwendung eines Ladungstransportmaterials, das aus einem Anilinderivat der Formel (1):

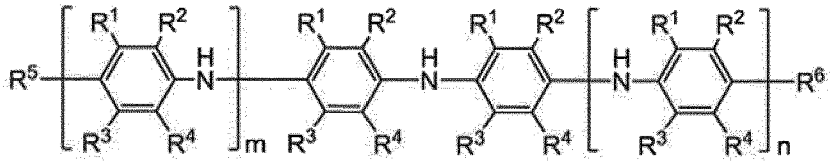

(1)

worin $R^1$ bis $R^6$ jeweils unabhängig ein Wasserstoffatom, ein Halogenatom, eine Nitrogruppe, eine Cyanogruppe, eine Aminogruppe, eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen, die gegebenenfalls mit $Z^1$ substituiert sind, eine Arylgruppe mit 6 bis 20 Kohlenstoffatomen oder eine Heteroarylgruppe mit 2 bis 20 Kohlenstoffatomen sind, die gegebenenfalls mit $Z^2$, $-NHY^1$, $-NY^2Y^3$, $-OY^4$ oder $-SY^5$ substituiert sind;
$Y^1$ bis $Y^5$ jeweils unabhängig eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen, die gegebenenfalls mit $Z^1$ substituiert sind, oder eine Arylgruppe mit 6 bis 20 Kohlenstoffatomen oder eine Heteroarylgruppe mit 2 bis 20 Kohlenstoffatomen sind, die gegebenenfalls mit $Z^2$ substituiert sind;
$Z^1$ ein Halogenatom, eine Nitrogruppe, eine Cyanogruppe, eine Aminogruppe oder eine Arylgruppe mit 6 bis 20 Kohlenstoffatomen oder eine Heteroarylgruppe mit 2 bis 20 Kohlenstoffatomen ist, die gegebenenfalls mit $Z^3$ substituiert sind;
$Z^2$ ein Halogenatom, eine Nitrogruppe, eine Cyanogruppe, eine Aminogruppe oder eine Alkylgruppe mit 1 bis 20 Kohlenstoffatomen, eine Alkenylgruppe mit 2 bis 20 Kohlenstoffatomen oder eine Alkinylgruppe mit 2 bis 20 Kohlenstoffatomen ist, die gegebenenfalls mit $Z^3$ substituiert sind; und
$Z^3$ ein Halogenatom, eine Nitrogruppe, eine Cyanogruppe oder eine Aminogruppe ist; und
m und n jeweils unabhängig eine ganze Zahl von 1 bis 5 sind, und
einem aus Wolframatophosphorsäure bestehenden Dotiermaterial besteht,
wobei das Verhältnis $W_D/W_H$ zwischen der Masse $W_D$ des Dotiermaterials und der Masse $W_H$ des Ladungstransportmaterials die folgende Beziehung erfüllt:

$$3{,}5 \leq W_D/W_H \leq 15{,}0;$$

und
wobei das Molekulargewicht des Anilinderivats 300 bis 5000 beträgt.

5. Ladungstransportlack nach Anspruch 1 oder 2 oder Ladungstransportdünnfilm nach Anspruch 3 oder 4, wobei das Molekulargewicht des Anilinderivats 3000 oder weniger beträgt.

6. Elektronisches Gerät, umfassend einen Ladungstransportdünnfilm nach Anspruch 3 oder 4.

7. Organische Elektrolumineszenzvorrichtung, umfassend einen Ladungstransportdünnfilm nach Anspruch 3 oder 4.

8. Organische Elektrolumineszenzvorrichtung nach Anspruch 7, wobei der Ladungstransportdünnfilm eine Lochinjektionsschicht oder eine Lochtransportschicht ist.

9. Verfahren zur Herstellung eines Ladungstransportdünnfilms, **dadurch gekennzeichnet, dass** ein Ladungstransportlack nach Anspruch 1 oder 2 auf ein Substrat aufgebracht und das Lösungsmittel abgedampft wird.

10. Verfahren zur Herstellung eines Ladungstransportdünnfilms nach Anspruch 9, wobei das Lösungsmittel durch Backen des aufgebrachten Lacks unter Stickstoffatmosphäre abgedampft wird.

11. Verfahren zur Herstellung eines Ladungstransportdünnfilms nach Anspruch 9, wobei das Lösungsmittel durch Backen des aufgebrachten Lacks im Vakuum abgedampft wird.

12. Verfahren zur Herstellung einer organischen Elektrolumineszenzvorrichtung unter Verwendung eines Ladungstransportdünnfilms nach Anspruch 3 oder 4.

13. Verfahren zur Herstellung einer organischen Elektrolumineszenzvorrichtung unter Verwendung des durch ein Herstellungsverfahren nach einem der Ansprüche 9 bis 11 erhaltenen Ladungstransportdünnfilms.

## Revendications

1. Vernis de transport de charge **caractérisé en ce qu'**il comprend un matériau de transport de charge composé d'un dérivé d'aniline de formule (1)

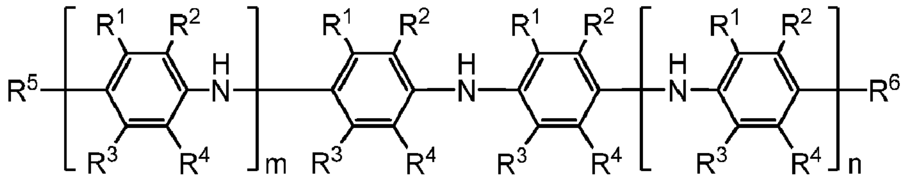

(1)

dans laquelle chacun de $R^1$ à $R^6$ est indépendamment un atome d'hydrogène, un atome d'halogène, un groupe nitro, un groupe cyano, un groupe amino, un groupe alkyle de 1 à 20 carbones, un groupe alcényle de 2 à 20 carbones ou un groupe alcynyle de 2 à 20 carbones qui peut être substitué par $Z^1$, un groupe aryle de 6 à 20 carbones ou un groupe hétéroaryle de 2 à 20 carbones qui peut être substitué par $Z^2$, $-NHY^1$, $-NY^2Y^3$, $-OY^4$ ou $-SY^5$ ;
chacun de $Y^1$ à $Y^5$ est indépendamment un groupe alkyle de 1 à 20 carbones, un groupe alcényle de 2 à 20 carbones ou un groupe alcynyle de 2 à 20 carbones qui peut être substitué par $Z^1$, ou un groupe aryle de 6 à 20 carbones ou un groupe hétéroaryle de 2 à 20 carbones qui peut être substitué par $Z^2$ ;
$Z^1$ est un atome d'halogène, un groupe nitro, un groupe cyano, un groupe amino, ou un groupe aryle de 6 à 20 carbones ou un groupe hétéroaryle de 2 à 20 carbones qui peut être substitué par $Z^3$ ;
$Z^2$ est un atome d'halogène, un groupe nitro, un groupe cyano, un groupe amino, ou un groupe alkyle de 1 à 20 carbones, un groupe alcényle de 2 à 20 carbones ou un groupe alcynyle de 2 à 20 carbones qui peut être substitué par $Z^3$ ; et
$Z^3$ est un atome d'halogène, un groupe nitro, un groupe cyano ou un groupe amino ; et
chacun de m et n est indépendamment un entier de 1 à 5,
un matériau dopant consistant en acide phosphotungstique, et un solvant organique,
dans lequel le rapport $W_D/W_H$ de la masse $W_D$ du matériau dopant à la masse $W_H$ du matériau de transport de charge satisfait à la relation

$$3{,}5 \leq W_D/W_H \leq 15{,}0 \ ;$$

et
dans lequel la masse moléculaire du dérivé d'aniline est de 300 à 5000.

2. Vernis de transport de charge selon la revendication 1, dans lequel
chacun de $R^1$ à $R^6$ est indépendamment un atome d'hydrogène, un atome d'halogène, un groupe alkyle de 1 à 20 carbones qui peut être substitué par $Z^1$, un groupe aryle de 6 à 20 carbones qui peut être remplacé par $Z^2$, $-NHY^1$,

$-NY^2Y^3$, $OY^4$ ou $-SY^5$ ;
chacun de $Y^1$ à $Y^5$ est indépendamment un groupe alkyle de 1 à 10 carbones qui peut être substitué par $Z^1$ ou un groupe aryle de 6 à 10 carbones qui peut être substitué par $Z^2$ ;
$Z^1$ est un atome d'halogène ou un groupe aryle de 6 à 10 carbones qui peut être substitué par $Z^3$ ; et
$Z^2$ est un atome d'halogène ou un groupe alkyle de 1 à 10 carbones qui peut être substitué par $Z^3$.

**3.** Film mince de transport de charge produit par utilisation du vernis de transport de charge selon la revendication 1 ou 2.

**4.** Film mince de transport de charge produit par utilisation d'un matériau de transport de charge composé d'un dérivé d'aniline de formule (1)

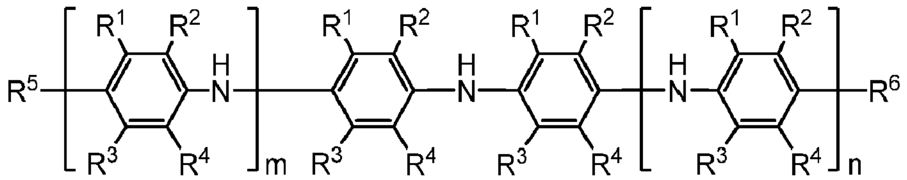

(1)

dans laquelle chacun de $R^1$ à $R^6$ est indépendamment un atome d'hydrogène, un atome d'halogène, un groupe nitro, un groupe cyano, un groupe amino, un groupe alkyle de 1 à 20 carbones, un groupe alcényle de 2 à 20 carbones ou un groupe alcynyle de 2 à 20 carbones qui peut être substitué par $Z^1$, un groupe aryle de 6 à 20 carbones ou un groupe hétéroaryle de 2 à 20 carbones qui peut être substitué par $Z^2$, $-NHY^1$, $-NY^2Y^3$, $-OY^4$ ou $-SY^5$ ;
chacun de $Y^1$ à $Y^5$ est indépendamment un groupe alkyle de 1 à 20 carbones, un groupe alcényle de 2 à 20 carbones ou un groupe alcynyle de 2 à 20 carbones qui peut être substitué par $Z^1$, ou un groupe aryle de 6 à 20 carbones ou un groupe hétéroaryle de 2 à 20 carbones qui peut être substitué par $Z^2$ ;
$Z^1$ est un atome d'halogène, un groupe nitro, un groupe cyano, un groupe amino, ou un groupe aryle de 6 à 20 carbones ou un groupe hétéroaryle de 2 à 20 carbones qui peut être substitué par $Z^3$ ;
$Z^2$ est un atome d'halogène, un groupe nitro, un groupe cyano, un groupe amino, ou un groupe alkyle de 1 à 20 carbones, un groupe alcényle de 2 à 20 carbones ou un groupe alcynyle de 2 à 20 carbones qui peut être substitué par $Z^3$ ; et
$Z^3$ est un atome d'halogène, un groupe nitro, un groupe cyano ou un groupe amino ; et
chacun de m et n est indépendamment un entier de 1 à 5,
un matériau dopant consistant en acide phosphotungstique,
dans lequel le rapport $W_D/W_H$ de la masse $W_D$ du matériau dopant à la masse $W_H$ du matériau de transport de charge satisfait à la relation

$$3,5 \leq W_D/W_H \leq 15,0 ;$$

et
dans lequel la masse moléculaire du dérivé d'aniline est de 300 à 5000.

**5.** Vernis de transport de charge selon la revendication 1 ou 2, ou film mince de transport de charge selon la revendication 3 ou 4, dans lequel la masse moléculaire du dérivé d'aniline est de 3000 ou moins.

**6.** Dispositif électronique comprenant le film mince de transport de charge de la revendication 3 ou 4.

**7.** Dispositif électroluminescent organique comprenant le film mince de transport de charge de la revendication 3 ou 4.

**8.** Dispositif électroluminescent organique selon la revendication 7, dans lequel le film mince de transport de charge est une couche d'injection de trou ou une couche de transport de trou.

**9.** Procédé pour produire un film mince de transport de charge, **caractérisé par** l'application du vernis de transport de charge de la revendication 1 ou 2 sur un substrat et l'élimination du solvant par évaporation.

**10.** Procédé pour produire un film mince de transport de charge selon la revendication 9, dans lequel le solvant est éliminé par évaporation au moyen d'une cuisson du vernis appliqué dans une atmosphère d'azote.

**11.** Procédé pour produire un film mince de transport de charge selon la revendication 9, dans lequel le solvant est éliminé par évaporation au moyen d'une cuisson du vernis appliqué sous vide.

**12.** Procédé pour produire un dispositif électroluminescent organique utilisant le film mince de transport de charge de la revendication 3 ou 4.

**13.** Procédé pour produire un dispositif électroluminescent organique utilisant le film mince de transport de charge obtenu par le procédé de production de l'une quelconque des revendications 9 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002151272 A
- WO 2010058777 A